(19) Europäisches Patentamt
European Patent Office
Office européen des brevets

(11) Veröffentlichungsnummer: **0 045 429**
**B1**

(12) # EUROPÄISCHE PATENTSCHRIFT

(45) Veröffentlichungstag der Patentschrift: **31.08.83**

(51) Int. Cl.³: **C 07 C 45/42**, C 07 C 47/542, C 07 C 47/55

(21) Anmeldenummer: **81105680.3**

(22) Anmeldetag: **20.07.81**

(54) **Verfahren zur Herstellung von 4-tert.-Butylbenzaldehyden.**

(30) Priorität: **01.08.80 DE 3029367**

(43) Veröffentlichungstag der Anmeldung: **10.02.82 Patentblatt 82/6**

(45) Bekanntmachung des Hinweises auf die Patenterteilung: **31.08.83 Patentblatt 83/35**

(84) Benannte Vertragsstaaten: **BE CH DE FR GB IT LI**

(56) Entgegenhaltungen:
**EP-A-0 011 281**
**FR-A-2 163 741**
**GB-A-816 253**

**CHEMICAL ABSTRACTS, Band 87, Nr. 11, 12. September 1977, Seite 564, Nr. 84685j Columbus, Ohio, U.S.A.**

(73) Patentinhaber: **BAYER AG, Zentralbereich Patente, Marken und Lizenzen, D-5090 Leverkusen 1, Bayerwerk (DE)**

(72) Erfinder: **Blank, Heinz Ulrich, Dr., Am Geusfelde 35, D-5068 Odenthal (DE)**
Erfinder: **Wolters, Erich, Dr., Streffenweg 22, D-5162 Niederzier (DE)**

EP 0 045 429 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 4-tert.-Butylbenzaldehyden

Die Erfindung betrifft ein Verfahren zur Herstellung von gegebenenfalls substituierten 4-tert.-Butylbenzaldehyden aus gegebenenfalls substituierten 4-tert.-Butyltoluolen durch Halogenierung und anschliessende Verseifung des Halogenierungsgemisches.

Es ist aus der US-PS 2 158 519 (Beispiel 2) bekannt, 4-tert.-Butylbenzaldehyd durch Umsetzung von Benzol mit Kohlenstoffmonoxid und tert.-Butylchlorid in Gegenwart von Aluminiumchlorid und Kupferchlorid herzustellen, wobei allerdings keine Ausbeuten angegeben werden.

Wie Vergleichsversuche zeigen, erhält man den 4-tert.-Butylbenzaldehyd nur in Ausbeuten von unter 30% der Theorie.

Weiterhin ist aus der US-PS 3 833 660 ein Verfahren zur Herstellung von 4-tert.-Butylbenzaldehyd (vgl. Tabelle I) bekannt, wonach tert.-Butylbenzol mit Hexamethylentetramin in Gegenwart von Trifluoressigsäure umgesetzt und anschliessend hydrolysiert wird. Die Ausbeute an 4-tert.-Butylbenzaldehyd wird mit 75% angegeben.

Nachteilig bei diesem Verfahren ist die unbefriedigende Ausbeute an 4-tert.-Butylbenzaldehyd und die umständliche und damit unwirtschaftliche Aufarbeitung des Reaktionsgemisches.

Ausserdem werden bei diesem Verfahren relativ grosse Mengen an Hexamethylentetramin und Trifluoressigsäure benötigt. Dadurch wird die Wirtschaftlichkeit des Verfahrens zusätzlich beeinträchtigt.

Aus der DE-OS 28 49 692 ist ausserdem ein Verfahren zur Herstellung von p-tert.-Butylbenzaldehyd und dessen am Kern durch Halogen substituierten Derivaten bekannt, das dadurch gekennzeichnet ist, dass man p-tert.-Butyltoluol und dessen am Kern durch Halogen substituierte Derivate mit etwa 2 Mol Brom pro Mol organisches Ausgangsmaterial bei Temperaturen von etwa 40 bis 200°C, gegebenenfalls unter der Einwirkung energiereicher Strahlung oder in Gegenwart von Radikalbildnern umsetzt und das dabei gebildete p-tert.-Butylbenzalbromid und dessen am Kern durch Halogen substituierte Derivate gegebenenfalls nach deren Isolierung bei erhöhter Temperatur, gegebenenfalls in Gegenwart von üblichen Verseifungskatalysatoren, verseift.

Wie der DE-OS auf Seite 9, letzter Absatz, zu entnehmen ist, beschränkt sich das Verfahren der DE-OS auf die Bromierung von p-tert.-Butyltoluol, da bei der Halogenierung mit Chlor neben der Chlorierung der kernständigen Methylgruppe der p-tert.-Butyltoluole auch eine beträchtliche Kernchlorierung der Butyltoluole stattfindet.

Wie ausserdem eigene Versuche zeigen, findet in erheblichem Masse unter bestimmten Chlorierungsbedingungen eine Substitution der Wasserstoffatome der tert.-Butylgruppe statt.

Von Nachteil ist bei dem in der DE-OS beschriebenen Verfahren, dass das für die Bromierung verwendete Brom relativ teuer ist, was die Wirtschaftlichkeit des Verfahrens beeinträchtigt.

Es wurde nun ein Verfahren zur Herstellung von gegebenenfalls am Kern durch Fluor, Chlor, Brom oder Jod ein- oder mehrfach substituiertem 4-tert.-Butylbenzaldehyd gefunden, das dadurch gekennzeichnet ist, dass man gegebenenfalls am Kern durch Fluor, Chlor, Brom oder Jod ein- oder mehrfach substituiertes 4-tert.-Butyltoluol mit 1,6 bis 2,4 Mol Bromchlorid, gegebenenfalls in Gegenwart von Brom und/oder Chlor, als Halogenierungsmittel, pro Mol gegebenenfalls substituiertem 4-tert.-Butyltoluol, bei Temperaturen von 40 bis 230°C, gegebenenfalls in Gegenwart von inerten organischen Lösungs- oder Verdünnungsmitteln, umsetzt und anschliessend das Halogenierungsgemisch, gegebenenfalls nach vorheriger Entfernung des im Gemisch enthaltenen, gegebenenfalls substituierten 4-tert.-Butylbenzylhalogenids, verseift.

Als Ausgangsprodukte für das erfindungsgemässe Verfahren dienen 4-tert.-Butyltoluol und dessen am Kern durch Fluor, Chlor, Brom oder Jod ein- oder mehrfach substituierte Derivate. Die monosubstituierten Derivate können durch folgende allgemeine Formel (I) wiedergegeben werden:

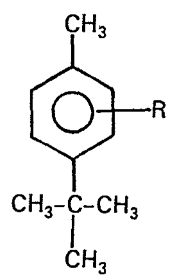

(I),

worin R für Fluor, Chlor, Brom oder Jod steht.

Bevorzugt wird 4-tert.-Butyltoluol in das erfindungsgemässe Verfahren eingesetzt.

Nach dem erfindungsgemässen Verfahren werden bevorzugt 1,8 bis 2,3 Mol Halogenierungsmittel pro Mol Ausgangsprodukt eingesetzt.

Dabei kann durch die jeweils eingesetzte Menge an Halogenierungsmittel die Zusammensetzung des Halogenierungsgemisches hinsichtlich des Gehaltes an Benzyl-, Benzal-, und Benzotrihalogenid variiert werden.

So kann nach dem erfindungsgemässen Verfahren bei vollständigem Umsatz des Ausgangsproduktes durch Einsatz von etwa 2,0 bis 2,1 Mol Halogenierungsmittel pro Mol Ausgangsprodukt ein Halogenierungsgemisch erhalten werden, das das gegebenenfalls substituierte 4-tert.-Butylbenzalhalogenid in einer Menge von etwa 90 bis 93 Gew.-% enthält. Das gegebenenfalls substituierte 4-tert.-Butylbenzylhalogenid und 4-tert.-Butylbenzotrihalogenid werden dabei nur in untergeordneten Mengen von ca. 2 bis 3 Gew.-% bzw. 3 bis 5 Gew.-% gebildet.

Bei Einsatz von etwa 1,8 bis 1,9 Mol Halogenierungsmittel pro Mol Ausgangsprodukt und vollständigem Umsatz des Ausgangsproduktes ist es möglich, ein Halogenierungsprodukt zu erhalten, das ca. 80 bis 85 Gew.-% gegebenenfalls substituiertes 4-tert.-Butylbenzalhalogenid neben ca. 15 bis 20 Gew.-% gegebenenfalls substituiertem 4-tert.-Butylbenzylhalogenid enthält.

Zur Erhöhung der Ausbeute an gegebenenfalls substituiertem 4-tert.-Butylbenzalhalogenid kann man einen Teil des Halogenierungsgemisches, das gegebenenfalls substituiertes 4-tert.-Butylbenzylhalogenid enthält, durch z.B. Destillation abtrennen und das gegebenenfalls substituierte 4-tert.-Butylbenzylhalogenid enthaltende Destillat zur weiteren Halogenierung wieder in das Reaktionsgemisch zurückführen.

Durch diese Verfahrensvariante gelingt es, die Ausbeute an gegebenenfalls substituiertem 4-tert.-Butylbenzalhalogenid zu steigern, ohne dass nennenswerte Mengen an gegebenenfalls substituiertem 4-tert.-Butylbenzotrihalogenid gebildet werden.

Weiterhin ist es möglich, 1,0 Mol gegebenenfalls substituiertes 4-tert.-Butyltoluol mit etwa 2,2 bis 2,4 Mol Halogenierungsmittel umzusetzen. Dabei wird ein Halogenierungsgemisch erhalten, das nur noch sehr wenig, nämlich ca. 0,1 bis 0,4 Gew.-% gegebenenfalls substituiertes 4-tert.-Butylbenzylhalogenid enthält. Dagegen wird im verstärkten Masse gegebenenfalls substituiertes 4-tert.-Butylbenzotrihalogenid gebildet (ca. 15 Gew.-%), neben ca. 85 Gew.-% gegebenenfalls substituiertem 4-tert.-Butylbenzalhalogenid.

Das in den Halogenierungsgemischen enthaltene 4-tert.-Butylbenzalhalogenid enthält etwa 5 bis 30 Gew.-%, vorzugsweise 10 bis 25 Gew.-%, besonders bevorzugt 20 Gew.-%, eines Benzalhalogenids der Formel (II)

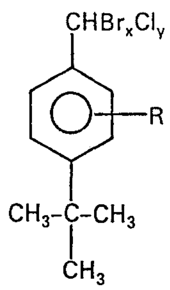

(II),

worin

x 0 oder 1 und y 1 oder 2 sein kann, jedoch die Summe x und y = 2 ist, bevorzugt

x 1 und y 1 ist, und

R Wasserstoff, Fluor, Chlor, Brom oder Jod bedeutet.

Der Rest besteht aus gegebenenfalls substituiertem 4-tert.-Butylbenzalbromid.

Das im Halogenierungsgemisch enthaltene, gegebenenfalls substituierte 4-tert.-Butylbenzylhalogenid enthält überwiegend (ca. 70 bis 95 Gew.-%) die entsprechende Bromverbindung und zu einem geringeren Anteil (ca. 5 bis 30 Gew.-%) die entsprechende Chlorverbindung.

Das im Halogenierungsgemisch enthaltene 4-tert.-Butylbenzotrihalogenid enthält in untergeordnetem Masse (ca. 5 bis 30 Gew.-%) 4-tert.-Butylbenzotrihalogenid der Formel (III)

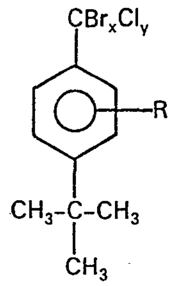

(III),

worin

x 1 oder 2 und y 1 oder 2 sein kann, jedoch die Summe x und y = 3 ist, und

R Wasserstoff, Fluor, Chlor, Brom oder Jod bedeutet.

In überwiegendem Masse (ca. 70 bis 95 Gew.-%) besteht das 4-tert.-Butylbenzotrihalogenid aus der entsprechenden Bromverbindung.

Die Halogenierung wird bevorzugt bei Temperaturen von 100 bis 200°C, besonders bevorzugt bei 160 bis 190°C durchgeführt.

Als organische Lösungs- oder Verdünnungsmittel können solche in das erfindungsgemässe Verfahren eingesetzt werden, die sich unter den Reaktionsbedingungen inert verhalten.

Zum Beispiel können halogenierte aliphatische Kohlenwasserstoffe mit 1 bis 5 Kohlenstoffatomen, bevorzugt 1 bis 2 Kohlenstoffatomen, wie Dichlormethan, Trichlormethan, Tetrachlormethan, Dichlorethan, Trichlorethan, Tetrachlorethan, Hexachlorethan, bevorzugt Tetrachlorethan, sowie gegebenenfalls halogenierte aromatische Kohlenwasserstoffe, wie Benzol, Brombenzol, Chlorbenzol, Dichlorbenzol, bevorzugt Dichlorbenzol, in das erfindungsgemässe Verfahren eingesetzt werden.

Die optimale Menge an einzusetzenden Lösungs- oder Verdünnungsmitteln hängt u.a. von der Reaktionstemperatur ab und kann leicht durch Vorversuche ermittelt werden.

Im allgemeinen werden bis zu 1000 Vol.-%, bevorzugt 5 bis 50 Vol.-%, bezogen auf das Ausgangsprodukt, eines inerten organischen Lösungs- oder Verdünnungsmittels in das erfindungsgemässe Verfahren eingesetzt. Vorteilhaft ist es, wenn das organische Lösungs- oder Verdünnungsmittel in wasserfreier Form verwendet wird.

Die inerten organischen Lösungs- oder Verdünnungsmittel können allein oder im Gemisch untereinander in das erfindungsgemässe Verfahren eingesetzt werden.

Bei Verwendung organischer Lösungs- oder Verdünnungsmittel kann es aber auch vorteilhaft sein, in Gegenwart von Wasser in einem 2-Phasensystem zu arbeiten, wobei die Wasserphase z.B. durch Zusatz von Alkali- oder Erdalkaliverbindungen, wie den Oxiden, Hydroxiden, Carbonaten, Hydrogencarbonaten, von Lithium, Natrium, Kalium, Magnesium oder Calcium sowie den entsprechenden Aluminiumverbindungen teilweise oder vollständig und gleichzeitig mit der Halogenierung neutralisiert wird (etwa pH 3-9, vorzugsweise pH 6 bis 7). Diese Ausführungsform der Reaktion erfolgt vorzugsweise bei etwa 40 bis 80°C, beispielsweise unter Siedebedingungen, sowie vorzugsweise unter Verwendung von Radikalbildnern wie Azo-bis-iso-buttersäure-nitril oder unter Bestrahlung mit natürlichem oder künstlichem Licht.

Das erfindungsgemässe Verfahren wird allerdings vorzugsweise ohne Lösungs- oder Verdünnungsmittel durchgeführt.

Ein besonderes Merkmal des erfindungsgemässen Verfahrens ist es, dass die Halogenierung wasserfrei ohne Verwendung von Radikalbildnern, Katalysatoren oder sonstigen Hilfsstoffen und ohne die Mitwirkung von natürlichem oder künstlichem Licht durchgeführt werden kann.

Es ist selbstverständlich auch möglich, das erfindungsgemässe Verfahren unter Verwendung von Radikalbildnern, Katalysatoren oder sonstigen Hilfsstoffen und unter Einwirkung von natürlichem oder künstlichem Licht durchzuführen.

In das erfindungsgemässe Verfahren kann sowohl reines Ausgangsprodukt als auch technisches Ausgangsprodukt mit einem Gehalt von ca. 95 bis 98 Gew.-% an gegebenenfalls substituiertem 4-tert.-Butyltoluol neben ca. 2 bis 3 Gew.-% Isomeren, gegebenenfalls substituierten tert.-Butyltoluolen, wie gegebenenfalls substituiertes 3-tert.-Butyltoluol, geringe Mengen an gegebenenfalls substituiertem Toluol sowie Spuren verschiedener anderer Verbindungen, wie Oligomere des Isobutens, eingesetzt werden.

Es ist auch möglich, stärker verunreinigtes, gegebenenfalls substituiertes 4-tert.-Butyltoluol, das etwa 1 bis 5 Gew.-% Oligomere des Isobutens neben diversen anderen Verbindungen, wie Spuren von Schwefelsäure, Di-tert.-Butylether, Mono- und Di-tert.-Butylsulfat, enthält, ohne Nachteile in das erfindungsgemässe Verfahren einzusetzen. So ist es z.B. möglich, ein aus der Alkylierung von Toluol stammendes undestilliertes 4-tert.-Butyltoluol, das gegebenenfalls von überschüssigem Toluol befreit wurde, in das erfindungsgemässe Verfahren einzusetzen.

Nachdem die Halogenierung beendet ist, wird in einer bevorzugten Arbeitsweise zu dem gegebenenfalls auf etwa 20 bis etwa 150°C, bevorzugt 50 bis 120°C, abgekühlten Reaktionsgemisch, gegebenenfalls nach Abtrennung von inerten organischen Lösungs- oder Verdünnungsmitteln, Ameisensäure gegeben und so lange gerührt, bis keine Gasentwicklung mehr zu beobachten ist. Im allgemeinen wird das Halogenierungsgemisch mit 0,3 bis 5,0 Mol, bevorzugt 0,7 bis 2,0 Mol, besonders bevorzugt 0,9 bis 1,4 Mol, Ameisensäure pro Mol Ausgangsprodukt umgesetzt.

Die Ameisensäure kann sowohl in konzentrierter Form als auch im Gemisch mit Wasser eingesetzt werden. Im allgemeinen wird eine 20 bis 100 gew.-%ige, bevorzugt eine 50 bis 95 gew.-%ige, besonders bevorzugt eine etwa 85 gew.-%ige, Ameisensäure eingesetzt.

Es ist auch möglich, die Verseifung des Halogenierungsgemisches in Gegenwart von Katalysatoren, wie Schwefelsäure oder den Chloriden oder Bromiden der Metalle der II., VII., VIII. Nebengruppe sowie der III. und V. Hauptgruppe des Periodensystems, durchzuführen.

Bevorzugt wird die Verseifung ohne Verwendung eines Katalysators durchgeführt.

Selbstverständlich ist es auch möglich, das Halogenierungsgemisch mit anderen üblichen Verseifungsmitteln, wie wässrige Chlor- oder Bromwasserstoffsäuren, wässriger Schwefelsäure oder mit Wasser und Zinkchlorid zu verseifen. Ebenso ist eine Verseifung unter neutralen bis schwach alkalischen Bedingungen, beispielsweise in Wasser bei pH 5 bis 8, vorzugsweise pH 6 bis 7, möglich. Als Basen werden vorzugsweise Hydroxide, Carbonate oder Oxide der Alkali- oder Erdalkalimetalle, wie $MgO$, $MgCO_3$, $Mg(OH)_2$, $CaO$, $Ca(OH)_2$ und $CaCO_3$ eingesetzt.

Die Verseifung wird in diesem Fall üblicherweise bei Temperaturen von 70 bis 200°C bevorzugt bei 100 bis 150°C, und unter Druck (bis 20 bar) durchgeführt.

Es ist auch möglich, die Verseifung des Halogenierungsgemisches nach dem erfindungsgemässen Verfahren in Gegenwart von organischen Lösungs- oder Verdünnungsmitteln durchzuführen. Beispielsweise können als organische Lösungs- oder Verdünnungsmittel halogenierte Kohlenwasserstoffe mit 1 bis 5 Kohlenstoffatomen oder halogenierte oder alkylierte Aromaten dem Halogenierungsgemisch zugesetzt werden.

Beispielsweise werden als organische Lösungs- oder Verdünnungsmittel genannt: Tetrachlorethan, Dichlorethan, Trichlorethan, Hexachlorethan, Benzol, Chlorbenzol, Dichlorbenzol, Toluol, tert.-Butyltoluol.

Weiterhin ist es möglich, Verseifung in Gegenwart von Essigsäure und Propionsäure sowie in Gegenwart von Formiat, Acetat oder Propionat durchzuführen.

Die Menge der zuzusetzenden organischen Lösungs- oder Verdünnungsmittel beträgt im allgemeinen bis zu 300 Vol.-%, bevorzugt 10 bis 50 Vol.-%, bezogen auf eingesetztes 4-tert.-Butyltoluol.

Das erfindungsgemässe Verfahren wird beispielsweise so durchgeführt, dass man in vorgelegtes, gegebenenfalls substituiertes 4-tert.-Butyltoluol die berechnete Menge an Halogenierungsmittel bei einer Temperatur von 160 bis 190°C einträgt.

Das als Halogenierungsmittel verwendete Bromchlorid, auch als Chlorbrom oder Bromchlor bekannt, kann in Gegenwart von Brom und/oder Chlor verwendet werden. Das Halogenierungsmittel wird beispielsweise in flüssiger Form oder in gelöster Form in üblicher Weise über eine Dosiervorrichtung in das Reaktionsgemisch eingebracht, wobei darauf geachtet wird, dass die Dosierung in dem Masse erfolgt, wie das Halogenierungsmittel im Ansatz verbraucht wird, so dass gewöhnlich bei einer stationären Konzentration an Halogenierungsmittel von unter 5 Gew.-%, vorzugsweise unter 2 Gew.-% und insbesondere

unter 1 Gew.-%, gearbeitet wird. Ebenso ist es möglich, das Halogenierungsmittel vor dem Reaktor zu verdampfen und gasförmig in den Reaktor einzudosieren, wobei gegebenenfalls in Gegenwart eines Inertgases gearbeitet wird. Als Inertgase können beispielsweise Stickstoff, Chlorwasserstoff, Bromwasserstoff, Kohlendioxid und Schwefeldioxid verwendet werden. Vorzugsweise verwendet man Stickstoff. Das Halogenierungsmittel wird gewöhnlich durch Vermischen von Brom mit Chlor und/oder duch Vermischen von Bromwasserstoff mit Chlor hergestellt. Beispielsweise vermischt man Brom und/oder Bromwasserstoff mit Chlor im Verhältnis 10 Brom : 1 Chlor bis 1 Brom : 10 Chlor. Vorzugsweise enthält das Bromierungsmittel Brom und Chlor im Verhältnis 2:1 bis 1:2 und insbesondere im Verhältnis von etwa 1:1. Das Halogenierungsmittel kann in der Flüssig- oder Gasphase hergestellt werden, gegebenenfalls in Gegenwart eines gasförmigen oder flüssigen Verdünnungsmittels. Ein entsprechend hergestelltes gasförmiges bromchlorhaltiges Halogenierungsmittel kann direkt für das erfindungsgemässe Verfahren verwendet werden. Ebenso ist es möglich, ein solches Halogenierungsmittel durch Kühlung zu kondensieren oder in einem Lösungsmittel zu lösen. Auch können entsprechend hergestellte Bromchlor enthaltende Halogenierungsmittel, die in der Flüssigphase, gegebenenfalls in Gegenwart eines Lösungsmittels, hergestellt wurden, direkt für das erfindungsgemässe Verfahren verwendet werden.

Nach Ende der Halogenierung wird das Halogenierungsgemisch auf etwa 50 bis 70°C abgekühlt und mit der berechneten Menge Ameisensäure, bevorzugt einer 85%igen Ameisensäure, versetzt.

Das Gemisch wird dann bei 100 bis 130°C solange gerührt, bis keine Gasentwicklung mehr zu beobachten ist.

Die Aufarbeitung des Reaktionsgemisches kann so durchgeführt werden, dass man die flüchtigen Bestandteile, wie überschüssige Ameisensäure, im Vakuum durch Destillation entfernt.

Das erfindungsgemässe Verfahren kann sowohl kontinuierlich als auch diskontinuierlich durchgeführt werden.

Nach dem erfindungsgemässen Verfahren erhält man beispielsweise ein Rohöl, das etwa 91 bis 93 Gew.-% gegebenenfalls substituiertes 4-tert.-Butylbenzaldehyd neben geringen Mengen an gegebenenfalls substituiertem 4-tert.-Butylbenzxylhalogenid und/oder gegebenenfalls substituiertem 4-tert.-Butylbenzylalkohol sowie gegebenenfalls substituierte 4-tert.-Butylbenzoesäure enthält.

Die Ausbeute an gegebenenfalls substituiertem 4-tert.-Butylbenzaldehyd beträgt 88 bis 93% der Theorie, bezogen auf eingesetztes 4-tert.-Butyltoluol.

Der als Rohöl erhältliche 4-tert.-Butylbenzaldehyd kann als solcher ohne weitere Reinigung, z.B. für die Herstellung von Riechstoffen [Riechstoff Ind. 13, 81 (1938)] verwendet werden.

Selbstverständlich kann bei Bedarf das erhaltene Rohöl, z.B. durch Destillation, weiter aufgearbeitet werden. Man erhält dann die gegebenenfalls substituierten 4-tert.-Butylbenzaldehyde in reiner Form.

Die Vorteile des erfindungsgemässen Verfahrens liegen in den hohen Ausbeuten von über 88% der Theorie an gegebenenfalls substituiertem 4-tert.-Butylbenzaldehyd, der einfachen Durchführung der Reaktion, gegebenenfalls in nur einem Reaktionsgefäss (Eintopfverfahren), in der unkomplizierten Aufarbeitungsweise des Reaktionsgemisches und in dem um etwa 50% reduzierten Einsatz an Brom gegenüber dem Stand der Technik (DE-OS 28 49 692).

Weiterhin kann es vorteilhaft sein, aus gegebenenfalls anfallendem 4-tert.-Butylbenzotrihalogenid 4-tert.-Butylbenzoesäure herzustellen, die für die Herstellung von Alkydharzen und Lackrohstoffen verwendet wird (Roempp, 7. Auflage, Band 1, Seite 455).

Es ist ausserordentlich überraschend, dass nach dem erfindungsgemässen Verfahren die gegebenenfalls substituierten 4-tert.-Butylbenzaldehyde in solch guten Ausbeuten erhalten werden, da es aufgrund des Standes der Technik (DE-OS 28 49 692, Seite 4) zu erwarten war, dass die Seitenkettenhalogenierung mit dem erfindungsgemässen Halogenierungsmittel bei höheren Temperaturen wegen der eingangs aufgeführten Nebenreaktionen nur mässige Ausbeuten an Benzalhalogeniden liefern würde, wodurch sich zwangsläufig auch die Ausbeute an Aldehyden verringerte.

Ausserdem war zu erwarten, dass sich bei dem erfindungsgemässen Verfahren, das ja ohne Reinigung des Benzalhalogenids beispielsweise in einem sogenannten «Eintopfverfahren» durchgeführt werden kann, in grossen Mengen Nebenprodukte bildeten, die ebenfalls die Ausbeute an Aldehyd beeinträchtigen würden.

Die nachfolgenden Beispiele sollen das erfindungsgemässe Verfahren verdeutlichen, ohne es jedoch auf diese Beispiele einzuschränken.

Beispiel 1

In einem 500 ml Dreihalskolben mit Tropftrichter, dessen Eintropfrohr bis an den Boden des Kolbens reicht, werden 120,0 g 4-tert.-Butyltoluol (97%ig, 2% Isomere; 0,80 Mol) unter Rühren auf 160 bis 170°C aufgeheizt und unter Lichtausschluss im Verlauf von 6 Stunden 196,4 g Bromchlorid (1,7 Mol) so zugetropft, dass im Rückflusskühler kein Bromchlorid erscheint. Es wird noch ca. 10 Minuten bis zum Ende der Gasentwicklung nachgerührt.

Das Reaktionsgemisch erstarrt bei Raumtemperatur zu 237 g eines hellbraunen Kristallkuchens, der zu ca. 90% aus 4-tert.-Butylbenzalhalogenid, zu jeweils ca. 3% aus Benzylhalogenid und Benzotrihalogenid besteht.

Nachdem das Gemisch auf 70°C abgekühlt ist, werden 56,3 g 85%ige wässrige Ameisensäure (1,04 Mol Ameisensäure) hinzugefügt. Unter

Rühren wird nun die Temperatur des Reaktionsgemisches in dem Masse gesteigert, das die rege Gasentwicklung zulässt. Nach 10 bis 12 Stunden ist bei einer Temperatur von 110°C die Gasentwicklung beendet. Im Wasserstrahlvakuum werden 22,7 g Ameisensäure abdestilliert. Es bleiben 130,8 g eines Öls zurück, das 91,3% 4-tert.-Butylbenzaldehyd, 2,1% 4-tert.-Butylbenzylhalogenid, 5,1% 4-tert.-Butylbenzoesäure und 1,1% 3-tert.-Butylbenzaldehyd enthält.

Bei der Destillation dieses Öls werden erhalten: 118,4 g 4-tert.-Butylbenzaldehyd (96,8%ig mit 1,0% 3-tert.-Butylbenzaldehyd), 2,2% 4-tert.-Butylhalogenid und 11,2 g eines Rückstands, der 95,3% 4-tert.-Butylbenzoesäure und 2,8% 4-tert.-Butylbenzaldehyd enthält. Dies entspricht einer Ausbeute an 4-tert.-Butylbenzaldehyd von 91,4% bezogen auf eingesetztes 4-tert.-Butyltoluol.

Beispiel 2

In einem 2 l Dreihalskolben mit einem Tropftrichter, dessen Einleitungsrohr bis an den Kolbenboden reicht, werden 600 g 4-tert.-Butyltoluol (97%ig, 2% Isomere, 3,93 Mol) unter Rühren auf 160 bis 170°C aufgeheizt und im Verlauf von 6 bis 7 Stunden 980,3 g Bromchlorid (8,5 Mol) so zugetropft, dass im Rückflusskühler kein Bromchlorid erscheint. Anschliessend wird noch etwa 10 Minuten bis zu Beendigung der Gasentwicklung nachgerührt. Man lässt das Reaktionsgemisch auf 70°C abkühlen und fügt dann 217,0 g 85%ige wässrige Ameisensäure (4,01 Mol) hinzu. Unter Rühren wird nun die Temperatur des Reaktionsgemisches in dem Masse gesteigert, das die rege Gasentwicklung erlaubt. Nach ca. 12 Stunden ist bei einer Temperatur von 110°C die Gasentwicklung beendet.

Unter gutem Rühren wird nun mit ca. 1 l einer ca. 5%igen $Na_2CO_3$-Lösung auf pH = 8,5 gestellt. Die organische Phase wird abgetrennt. Die wässrige Phase liefert nach Ansäuern, Absaugen und Trocknen 42,2 g 4-tert.-Butylbenzoesäure (Fp.: 164°C). Das Gewicht der getrockneten organischen Phase beträgt 614,5 g und enthält 96,5% 4-tert.-Butylbenzaldehyd, 1,0% 3-tert.-Butylbenzaldehyd und 1,7% 4-tert.-Butylbenzylhalogenide. Dies entspricht einer Rohausbeute an 4-tert.-Butylbenzaldehyd von 93,1%, bezogen auf eingesetztes 4-tert.-Butyltoluol. Die Destillation ergibt 592,3 g 4-tert.-Butylbenzaldehyd (97,0%ig, 0,9% 3-tert.-Butylbenzaldehyd, 1,3% 4-tert.-Butylbenzylhalogenide) und 16,2 g eines Rückstandes, der ca. 20% 4-tert.-Butylbenzaldehyd, ca. 9% 4-tert.-Butylbenzylhalogenide und ca. 70% 4-tert.-Butylbenzoesäure enthält. Die isolierte Menge an 4-tert.-Butylbenzaldehyd entspricht einer Ausbeute von 90,2%, bezogen auf eingesetztes 4-tert.-Butyltoluol.

Beispiel 3

Apparatur und Ansatz wie im Beispiel 1 beschrieben.

Es werden jedoch 208,0 g Bromchlorid (1,8 Mol) eingesetzt. Das Reaktionsgemisch enthält 0,2% tert.-Butylbenzylhalogenide, 79,1% tert.-Butylbenzalhalogenide und 20,6% tert.-Butylbenzotrihalogenide. Wie im Beispiel 1 wird nun hydrolysiert und analog Beispiel 2 aufgearbeitet. Aus der wässrigen Phase werden 27,2 g 4-tert.-Butylbenzoesäure (Fp. 164 bis 165°C) erhalten. Dies entspricht 19,7% des eingesetzten 4-tert.-Butyltoluols. Aus der organischen Phase werden nach Destillation 99,8 g 4-tert.-Butylbenzaldehyd erhalten (99,1%ig, 0,8% 3-tert.-Butylbenzaldehyd, 0,1% 4-tert.-Butylbenzylhalogenid). Diese entspricht einer Ausbeute von 78,7% bezogen auf eingesetztes 4-tert.-Butyltoluol. Der Destillationsrückstand von 2,4 g enthält im wesentlichen 4-tert.-Butylbenzoesäure.

Beispiel 4

Apparatur und Ansatz wie im Beispiel 1 beschrieben.

Es werden jedoch nur 174,0 g (1,51 Mol) Bromchlorid eingesetzt. Das Reaktionsgemisch enthält 17,7% tert.-Butylbenzylhalogenid, 82,5% tert.-Butylbenzalhalogenide und 0,4% tert.-Butylbenzotrihalogenide. Über eine Kolonne werden im Vakuum bei einem Druck von 1 mbar 70,5 g des Reaktionsgemisches bis zu einer Kopftemperatur von 130°C abdestilliert. Es bleiben 154,0 g Reaktionsprodukt zurück, das 98,2% tert.-Butylbenzalhalogenid, 0,3% tert.-Butylbenzylhalogenid und 0,6% tert.-Butylbenzotrihalogenid enthält. Das Destillat, das neben tert.-Butylbenzalhalogenid 56,3% tert.-Butylbenzylhalogenid enthält, wird in die Halogenierung zurückgeführt. Der Destillationsrückstand wird analog Beispiel 1 hydrolysiert und destilliert. Man erhält 79,2 g 4-tert.-Butylbenzaldehyd (98,1%ig, 1,3% 3-tert.-Butylbenzaldehyd, 0,2% 4-tert.-Butylbenzylhalogenid). Der Destillationsrückstand von 1,9 g enthält im wesentlichen 4-tert.-Butylbenzoesäure.

**Patentansprüche**

1. Verfahren zur Herstellung von gegebenenfalls am Kern durch Fluor, Chlor, Brom oder Jod ein- oder mehrfach substituiertem 4-tert.-Butylbenzaldehyd, dadurch gekennzeichnet, dass man gegebenenfalls am Kern durch Fluor, Chlor, Brom oder Jod ein- oder mehrfach substituiertes 4-tert.-Butyltoluol mit 1,6 bis 2,4 Mol Bromchlorid, gegebenenfalls in Gegenwart von Brom und/oder Chlor, als Halogenierungsmittel, pro Mol gegebenenfalls substituiertem 4-tert.-Butyltoluol, bei Temperaturen von 40 bis 230°C, gegebenenfalls in Gegenwart von inerten organischen Lösungs- oder Verdünnungsmitteln, umsetzt und anschliessend das Halogenierungsgemisch, gegebenenfalls nach vorheriger Entfernung des im Gemisch enthaltenen, gegebenenfalls substituierten 4-tert.-Butylbenzylhalogenids, verseift.

2. Verfahren nach Anspruch 1, dadurch gekennzeichnet, dass man die Umsetzung mit 1,8 bis 2,3 Mol Halogenierungsmittel pro Mol Ausgangsprodukt durchführt.

3. Verfahren nach Ansprüchen 1 und 2, dadurch gekennzeichnet, dass man die Verseifung mit Ameisensäure durchführt.

4. Verfahren nach Ansprüchen 1 bis 3, dadurch gekennzeichnet, dass man die Verseifung mit 20 bis 100 gew.-%iger Ameisensäure durchführt.

5. Verfahren nach Ansprüchen 1 bis 3, dadurch gekennzeichnet, dass man die Verseifung mit 85 gew.-%iger Ameisensäure durchführt.

6. Verfahren nach Ansprüchen 1 bis 5, dadurch gekennzeichnet, dass man die Verseifung mit 0,3 bis 5 Mol Ameisensäure pro Mol Ausgangsprodukt durchführt.

7. Verfahren nach Ansprüchen 1 bis 5, dadurch gekennzeichnet, dass man die Verseifung mit 0,7 bis 2,0 Mol Ameisensäure pro Mol Ausgangsprodukt durchführt.

8. Verfahren nach Ansprüchen 1 bis 7, dadurch gekennzeichnet, dass man substituierte 4-tert.-Butyltoluole der Formel (I)

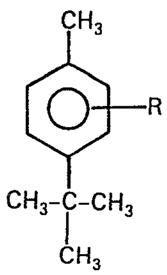

(I),

worin R für Fluor, Chlor, Brom oder Jod steht, einsetzt.

9. Verfahren nach Ansprüchen 1 bis 7, dadurch gekennzeichnet, dass man 4-tert.-Butyltoluol einsetzt.

**Claims**

1. Process for the preparation of 4-tert.-butylbenzaldehyde which is optionally monosubstituted or polysubstituted in the nucleus by fluorine, chlorine, bromine or iodine, characterised in that 4-tert.-butyltoluene which is optionally monosubstituted or polysubstituted in the nucleus by fluorine, chlorine, bromine or iodine is reacted with 1.6 to 2.4 mols of bromine chloride, if appropriate in the presence of bromine and/or chlorine, as the halogenating agent, per mol of optionally substituted 4-tert.-butyltoluene, at temperatures of 40 to 230°C, if appropriate in the presence of inert organic solvents or diluents, and the halogenation mixture is then hydrolysed, if appropriate after prior removal of the optionally substituted 4-tert.-butylbenzyl halide contained in the mixture.

2. Process according to Claim 1, characterised in that the reaction is carried out with 1.8 to 2.3 mols of halogenating agent per mol of starting material.

3. Process according to Claims 1 and 2, characterised in that the hydrolysis is carried out with formic acid.

4. Process according to Claims 1 to 3, characterised in that the hydrolysis is carried out with 20 to 100% strength by weight formic acid.

5. Process according to Claims 1 to 3, characterised in that the hydrolysis is carried out with 85% strength by weight formic acid.

6. Process according to Claims 1 to 5, characterised in that the hydrolysis is carried out with 0.3 to 5 mols of formic acid per mol of starting material.

7. Process according to Claims 1 to 5, characterised in that the hydrolysis is carried out with 0.7 to 2.0 mols of formic acid per mol of starting material.

8. Process according to Claims 1 to 7, characterised in that substituted 4-tert.-butyltoluenes of the formula (I)

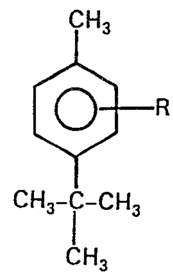

(I),

wherein R represents fluorine, chlorine, bromine or iodine, are employed.

9. Process according to Claims 1 to 7, characterised in that 4-tert.-butyltoluene is employed.

**Revendications**

1. Procédé de fabrication de 4-t-butylbenzaldéhyde éventuellement substituée une ou plusieurs fois sur le noyau par du fluor, du chlore, du brome ou de l'iode, caractérisé en ce qu'on fait réagir du 4-t-butyltoluène éventuellement substitué une ou plusieurs fois sur le noyau par du fluor, du chlore, du brome ou de l'iode avec 1,6 à 2,4 moles de chlorure de brome éventuellement en présence de brome et/ou de chlore, en tant qu'agent d'halogénation, par mole de 4-t-butyltoluène éventuellement substitué, à des températures de 40 à 230°C, éventuellement en présence de solvants ou diluants organiques inertes, puis en ce qu'on saponifie le mélange d'halogénation, éventuellement après élimination préalable de l'halogénure de 4-t-butylbenzyle éventuellement substitué contenu dans le mélange.

2. Procédé selon la revendication 1, caractérisé en ce qu'on exécute la réaction avec 1,8 à 2,3 moles d'agent d'halogénation par mole de produit de départ.

3. Procédé selon les revendications 1 et 2, caractérisé en ce qu'on effectue la saponification avec de l'acide formique.

4. Procédé selon les revendications 1 à 3, caractérisé en ce qu'on effectue la saponification avec de l'acide formique à 20–100% en poids.

5. Procédé selon les revendications 1 à 3, caractérisé en ce qu'on effectue la saponification avec de l'acide formique à 85% en poids.

6. Procédé selon les revendications 1 à 5, caractérisé en ce qu'on effectue la saponification avec 0,3 à 5 moles d'acide formique par mole de produit de départ.

7. Procédé selon les revendications 1 à 5, caractérisé en ce qu'on effectue la saponification avec 0,7 à 2,0 moles d'acide formique par mole de produit de départ.

8. Procédé selon les revendications 1 à 7, caractérisé en ce qu'on utilise des 4-t-butyltoluènes substitués de formule (I)

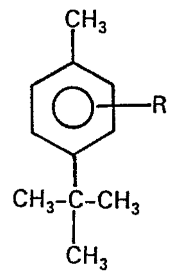

(I),

dans laquelle R représente du fluor, du chlore, du brome ou de l'iode.

9. Procédé selon les revendications 1 à 7, caractérisé en ce qu'on utilise du 4-t-butyltoluène.